# FASCICULE DE BREVET EUROPEEN

(11) **EP 1 303 251 B1**
(45) Date de publication et mention de la délivrance du brevet: **06.10.2004**
(21) Numéro de dépôt: 01943592.4
(22) Date de dépôt: 08.06.2001
(51) Int. Cl.: A61K 7/48

(54) **COMPOSITION, NOTAMMENT COSMETIQUE, RENFERMANT LA DHEA ET/OU UN PRECURSEUR OU DERIVE CHIMIQUE OU BIOLOGIQUE DE CELLE-CI, ET UN INHIBITEUR DE METALLOPROTEINASE**
ZUSAMMENSETZUNG, INSBENSODERE FÜR KOSMETIKA, ENTHALTEND DHEA UND/ODER EINEN CHEMISCHE ODER BIOLOGISCHE VORLÄUFER DAVON UND EINEN METALLOPROTEINASE INHIBITOR
COMPOSITION, IN PARTICULAR COSMETIC, CONTAINING DHEA AND/OR A CHEMICAL OR BIOLOGICAL PRECURSOR OR DERIVATIVE THEREOF, AND A METALLOPROTEINASE INHIBITOR

(30) Priorité: 13.07.2000 FR 0009218
(43) Date de publication de la demande: 23.04.2003
(73) Titulaire: L'OREAL, 75008 Paris (FR)
(72) Inventeur: BRETON, Lionel, F-78000 Versailles (FR)
(74) Mandataire: Renard, Emmanuelle
(86) Numéro de dépôt international: PCT/FR2001/001788
(87) Numéro de publication internationale: WO 2002/005775

(56) Documents cités:
- EP-A- 0 908 183
- EP-A- 1 090 628
- WO-A-98/36742
- WO-A-99/43329
- FR-A- 2 749 758
- US-A- 5 989 568
- US-A- 6 093 706
- KYU SUK LEE ET AL: "Effects of dehydroepiandrosterone on collagen and collagenase gene expression by skin fibroblasts in culture" JOURNAL OF DERMATOLOGICAL SCIENCE, vol. 23, juin 2000 (2000-06), pages 103-110, XP001004650

## Description

La présente invention se rapporte à une composition renfermant un précurseur de DHEA et au moins un inhibiteur de métalloprotéinase, ainsi qu'à l'utilisation de ladite composition, notamment pour prévenir ou traiter les signes de vieillissement cutané.

On constate chez la femme ménopausée un amincissement de la peau et/ou des muqueuses, dû notamment à des modifications au niveau du derme, principalement à une diminution du taux de collagène qui est particulièrement accentuée sous l'effet de certains facteurs tels que les rayons ultraviolets, la prise de certains médicaments tels que les corticoïdes ou la vitamine D, et le tabac. La peau présente alors une apparence molle et ridée, en contradiction avec les préoccupations actuelles de la population, visant à conserver le plus longtemps possible, ou à recouvrer, une peau d'aspect jeune.

On comprend donc l'importance qu'il y a à combattre la dégradation du collagène dans les tissus humains, pour ainsi lutter contre le vieillissement cutané et ses conséquences.

A cet effet, il a déjà été proposé d'utiliser la DHEA, ou déhydroépiandrostérone, pour remédier à l'atrophie du derme par inhibition de la perte de collagène et de tissu conjonctif (US-5,843,932). Il a en outre été décrit dans le brevet US-5,736,537 l'utilisation par voie orale d'esters de DHEA, en particulier du salicylate de DHEA, pour réguler l'atrophie de la peau due à un amincissement ou une dégradation générale du derme.

Par ailleurs, la Demanderesse a déjà démontré que la DHEA et/ou ses précurseurs ou dérivés, appliqués par voie topique, permettaient de prévenir ou lutter contre d'autres signes de vieillissement cutané, à savoir les taches pigmentaires (FR 99/12773), l'aspect papyracé de la peau (FR 00/00349), les rides, le relâchement cutané et le teint terne (EP-723 775).

Le document brevet WO99/43329 décrit une composition pour l'administration orale comprenant de la DHEA, qui peut être combinée à un anti-oxydant, parmi lesquels le lycopène est cité.

Or, il est apparu à la Demanderesse que l'association d'une sapogénine (ou d'un extrait naturel en contenant), qui est un précurseur de la DHEA, avec un inhibiteur de métalloprotéinase pouvait permettre de prévenir ou traiter plus efficacement les signes de vieillissement cutané, et notamment de prévenir ou traiter la perte de fermeté et/ou de souplesse de la peau et/ou l'atrophie de la peau et/ou la formation de rides.

La présente invention a donc pour objet une composition renfermant, dans un milieu cosmétiquement ou dermatologiquement acceptable, une sapogénine ou un extrait naturel en contenant, caractérisée en ce qu'elle comprend en outre au moins un inhibiteur de métalloprotéinase.

La DHEA a la formule (l) suivante :

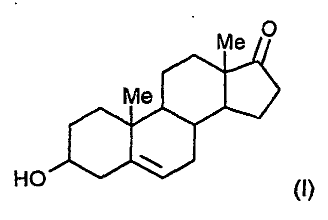

La DHEA utilisable selon l'invention est par exemple disponible auprès de la société AKZO NOBEL.

Par précurseurs de la DHEA, on entend ses précurseurs chimiques qui peuvent se transformer en DHEA par réaction chimique exogène. Des exemples de précurseurs chimiques sont les sapogénines tels que la diosgénine (ou spirost-5-èn-3-beta-ol), l'hécogénine, l'acétate d'hécogénine, le smilagénine et la sarsapogénine, ainsi que les extraits naturels en contenant, en particulier le fenugrec et les extraits de Dioscorées telles que la racine d'igname sauvage ou Wild Yam, sans que cette liste soit limitative.

Les précurseurs de DHEA seront désignés ci-dessous par "analogues de DHEA".

La concentration en analogues de DHEA dans la composition selon l'invention est avantageusement comprise entre 0,0001% et 10% en poids, de préférence entre 0,001% et 5% en poids, par rapport au poids total de la composition.

La composition selon la présente invention renferme, en association avec l'analogue de DHEA, au moins un inhibiteur de métalloprotéinase.

Par "inhibiteur de métalloprotéinase", on entend selon l'invention, toute molécule et/ou extrait végétai ou bactérien présentant une activité inhibitrice sur au moins l'une des métalloprotéinases exprimées et synthétisées par et dans la peau.

Les métalloprotéinases sont notamment décrites dans l'article de Y. HEROUY et al., European Journal of Dermatology, n° 3, vol. 10, Avril-Mai 2000, pp. 173-180.

La famille des métalloprotéinases est ainsi constituée de plusieurs groupes bien définis basés sur leurs ressemblances en terme de structure et de spécificité de substrat (voir Woessner J. F., Faseb Journal, vol. 5, 1991, 2145). Parmi ces groupes, on peut citer les collagénases destinées à dégrader les collagènes fibrillaires (MMP-1 ou collagénase interstitielle, MMP-8 ou collagénase de neutrophile, MMP-13 ou collagénase 3, MMP-18 ou collagénase 4), les gélatinases qui dégradent le collagène de type IV ou toute forme de collagène dénaturé (MMP-2 ou gélatinase A (72 kDa), MMP-9 ou gélatinase B (92 kDa)), les stromélysines (MMP-3 ou stromélysine 1, MMP-10 ou stromélysine 2, MMP-11 ou stromélysine 3) dont le large spectre d'activité s'adresse aux protéines de la matrice extracellulaire telles que les glycoprotéines (fibronectine, laminine), les protéoglycannes, etc., la matrilysine (MMP-7), la métalloélastase (MMP-12) ou encore les métalloprotéinases membranaires (MMP-14, MMP-15, MMP-16 et MMP-17).

Les métalloprotéinases (MMPs) sont les membres d'une famille d'enzymes protéolytiques (endoprotéases) qui possèdent un atome de zinc coordonné à 3 résidus cystéine et une méthionine dans leur site actif et qui dégradent les composants macromoléculaires de la matrice extracellulaire et des lames basales à pH neutre (collagène, élastine, etc ...). Très largement répandues dans le monde vivant, ces enzymes sont présentes, mais faiblement exprimées, dans des situations physiologiques normales comme la croissance des organes et le renouvellement des tissus. Leur surexpression chez l'homme et leur activation sont cependant liées à de nombreux processus qui impliquent la destruction et le remodelage de la matrice. Cela entraîne par exemple une résorption non contrôlée de la matrice extracellulaire.

Ainsi, l'exposition prolongée aux rayonnements ultraviolets, particulièrement aux rayonnements ultraviolets de type A et/ou B, a pour effet une stimulation de l'expression des collagénases, particulièrement de la MMP-1. C'est là une des composantes du vieillissement cutané photo-induit. En outre, on sait que l'activité des MMP-1, MMP-2 et MMP-9 augmente avec l'âge et que cette augmentation contribue, avec le ralentissement de la croissance cellulaire, au vieillissement chronologique de la peau (WO 98/36742).

Les métalloprotéinases sont produites et sécrétées sous une forme zymogène inactive (pro-enzyme). Ces formes zymogènes sont ensuite activées dans l'environnement extracellulaire par l'élimination d'une région propeptidique. Les membres de cette famille peuvent s'activer les uns les autres. La régulation de l'activité des MMPs se produit ainsi au niveau de l'expression des gènes (transcription et traduction), au niveau de l'activation de la forme zymogène, ou au niveau du contrôle local des formes actives.

Les principaux régulateurs de l'activité des MMPs sont les inhibiteurs tissulaires des métalloprotéinases ou TIMPs (tissue inhibitors of metalloproteinases). Cependant, l'expression des MMPs est également modulée par les facteurs de croissance, les cytokines, les produits oncogènes (ras, jun), ou encore les constituants matriciels.

Par inhibiteur de métalloprotéinase selon l'invention, on entend toute molécule capable de réguler l'activité des MMPs soit au niveau de l'expression des gènes (transcription et traduction), soit au niveau de l'activation de la forme zymogène des MMPs, soit encore au niveau du contrôle local des formes actives.

Ainsi, l'inhibiteur de métalloprotéinase selon l'invention peut être un inhibiteur tissulaire de métalloprotéinases (TIMP) tel que les peptides connus dans l'art antérieur sous les appellations TIMP-1, TIMP-2, TIMP-3 et TIMP-4 (Woessner J. F., Faseb Journal, 1991).

En variante, les inhibiteurs de métalloprotéinases convenant à une mise en oeuvre dans la présente invention peuvent être des inhibiteurs de MMP-1 d'origine naturelle ou synthétique. Par "origine naturelle", on entend l'inhibiteur de métalloprotéinase à l'état pur ou en solution à différentes concentrations, obtenu par différents procédés d'extraction à partir d'un élément, généralement une plante, d'origine naturelle. Par "origine synthétique", on entend l'inhibiteur de métalloprotéinase à l'état pur ou en solution à différentes concentrations, obtenu par synthèse chimique.

Selon une forme d'exécution particulièrement préférée de l'invention, on utilise un inhibiteur de métalloprotéinase d'origine naturelle, tel que le lycopène. Cet inhibiteur de métalloprotéinases a été décrit dans la demande de brevet FR 99/12507.

L'inhibiteur de métalloprotéinase représente de préférence de 10⁻¹² à 5%, et de préférence de 10⁻¹⁰ à 2%, du poids total de la composition. Bien entendu, si l'inhibiteur de métalloprotéinase est présent sous forme de solution contenant un extrait de plante, l'homme du métier saura ajuster la quantité de cette solution dans la composition selon l'invention, de façon à obtenir les gammes de concentrations ci-dessus en inhibiteur de métalloprotéinase.

La composition selon l'invention peut se présenter sous toutes les formes galéniques normalement utilisées pour une application topique sur la peau ou les cheveux, notamment sous forme d'une solution aqueuse ou huileuse, d'une émulsion huile-dans-eau ou eau-dans-huile ou multiple, d'une émulsion siliconée, d'une microémulsion ou nanoémulsion, d'un gel aqueux ou huileux, d'un produit anhydre liquide, pâteux ou solide, d'une dispersion d'huile dans une phase aqueuse en présence de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau.

En outre, dans la mesure où les inhibiteurs de métalloprotéinases sont déjà connus pour induire et/ou stimuler la croissance des cheveux ou des poils et/ou freiner leur chute (WO 99/58101), la Demanderesse pense -sans vouloir être liée par cette théorie- qu'ils seraient susceptibles de renforcer l'effet bénéfique des compositions capillaires à base de DHEA ou analogues, dans lesquelles la DHEA est elle-même connue pour agir sur la canitie (FR 99/12773).

Par suite, la composition selon l'invention peut en variante être une composition capillaire, se présentant notamment sous la forme de shampooing ou d'après-shampooing, par exemple.

De façon connue, la composition de l'invention peut contenir également les adjuvants habituels dans tes domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés, et représentent par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules. Ces adjuvants, ainsi que leurs concentrations, doivent être tels qu'ils ne nuisent pas aux propriétés avantageuses de l'association d'actifs selon l'invention.

Lorsque la composition selon l'invention est une émulsion, la proportion de la phase grasse peut aller de 5 à 80 % en poids, et de préférence de 5 à 50 % en poids par rapport au poids total de la composition. Les matières grasses, les émulsionnants et les coémulsionnants utilisés dans la composition sous forme d'émulsion sont choisis parmi ceux classiquement utilisés dans le domaine considéré. L'émulsionnant et le co-émulsionnant sont de préférence présents, dans la composition, en une proportion allant de 0,3 à 30 % en poids, et de préférence de 0,5 à 20 % en poids par rapport au poids total de la composition.

Comme matières grasses utilisables dans l'invention, on peut utiliser les huiles et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras tels que l'alcool cétylique, des acides gras, des cires et des gommes et en particulier les gommes de silicone.

Comme émulsionnants et coémulsionnants utilisables dans l'invention, on peut citer par exemple les esters d'acide gras et de polyéthylène glycol tels que le stéarate de PEG-100, le stéarate de PEG-50 et le stéarate de PEG-40 ; les esters d'acide gras et de polyol tels que le stéarate de glycéryle, le tristéarate de sorbitane et les stéarates de sorbitane oxyéthylénés disponibles sous les dénominations commerciales Tween® 20 ou Tween® 60, par exemple ; et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Comme actifs, on peut utiliser notamment les dépigmentants et les agents kératolytiques et/ou desquamants.

En cas d'incompatibilité, les actifs indiqués ci-dessus et/ou les analogues de DHEA et/ou l'inhibiteur de métalloprotéinase selon l'invention peuvent être incorporés dans des sphérules, notamment des vésicules ioniques ou non-ioniques et/ou des nanoparticules (nanocapsules et/ou nanosphères), de manière à les isoler les uns des autres dans la composition.

Selon une variante de l'invention, la composition peut être adaptée à une administration par voie orale. Dans ce cas, elle peut se présenter sous forme de sirops, de suspensions, de solutions, d'émulsions, de capsules, de granules ou de comprimés, par exemple.

Les doses quotidiennes d'analogues de DHEA administrées par voie orale peuvent être comprises entre 1 et 100 mg/jour, de préférence entre 25 et 75 mg/jour. Préférentiellement, l'analogue de DHEA est présent dans la composition selon l'invention en une quantité permettant son administration à une dose comprise entre 50 et 100 mg/jour, ladite posologie étant réalisée en une ou plusieurs prises, avec une dose unitaire préférentielle de 50 mg.

Dans tous les cas, la composition selon l'invention comprend une quantité efficace d'analogue de DHEA et d'inhibiteur de métalloprotéinase, suffisante pour obtenir l'effet recherché, et un milieu physiologiquement acceptable.

La composition selon l'invention trouve en particulier une application dans la prévention et le traitement des signes du vieillissement cutané.

La présente invention concerne donc également l'utilisation cosmétique de la composition selon l'invention pour prévenir ou traiter les signes de vieillissement cutané, en particulier pour prévenir ou traiter la perte de fermeté et/ou de souplesse de la peau et/ou l'atrophie de la peau et/ou la formation de rides.

Elle concerne aussi l'utilisation cosmétique de cette composition pour le traitement du cuir chevelu. Elle concerne enfin l'utilisation de la composition mentionnée ci-dessus pour fabriquer une préparation destinée au traitement du cuir chevelu.

## Revendications

1. Composition renfermant, dans un milieu cosmétiquement ou dermatologiquement acceptable, une sapogénine, ou un extrait naturel en contenant, et au moins un inhibiteur de métalloprotéinase.

2. Composition selon la revendication 1, **caractérisée en ce que** ladite sapogénine est choisie parmi la diosgénine, l'hécogénine, la smilagénine et la sarsapogénine.

3. Composition selon la revendication 1, **caractérisée en ce que** ledit extrait naturel est choisi parmi le fenugrec et les extraits de Dioscorées.

4. Composition selon la revendication 3, **caractérisée en ce que** ledit extrait de Dioscorée est un extrait de racine d'igname sauvage.

5. Composition selon l'une quelconque des revendications précédentes, **caractérisée en ce qu'**elle renferme de 0,001 à 5% en poids de sapogénine, par rapport au poids total de la composition.

6. Composition selon l'une quelconque des revendications précédentes, **caractérisée en ce que** ledit inhibiteur de métalloprotéinase est un inhibiteur tissulaire de métalloprotéinases.

7. Composition selon l'une quelconque des revendications 1 à 6, **caractérisée en ce que** ledit inhibiteur de métalloprotéinase est un inhibiteur de MMP-1 d'origine naturelle.

8. Composition selon la revendication 7, **caractérisée en ce que** ledit inhibiteur de métalloprotéinase est le lycopène.

9. Composition selon l'une quelconque des revendications précédentes, **caractérisée en ce qu'**elle comprend de 10⁻¹⁰ à 2% en poids d'inhibiteur de métalloprotéinase, par rapport au poids total de la composition.

10. Utilisation cosmétique de la composition selon l'une quelconque des revendications précédentes pour prévenir ou traiter les signes du vieillissement cutané.

11. Utilisation cosmétique de la composition selon l'une quelconque des revendications 1 à 9 pour prévenir ou traiter la perte de fermeté et/ou de souplesse de la peau et/ou l'atrophie de la peau et/ou la formation de rides.

12. Utilisation cosmétique de la composition selon l'une quelconque des revendications 1 à 9 pour le traitement du cuir chevelu.

13. Utilisation de la composition selon l'une quelconque des revendications 1 à 9 pour fabriquer une préparation destinée au traitement du cuir chevelu.

## Patentansprüche

1. Zusammensetzung, die in einem kosmetisch oder dermatologisch akzeptablen Medium ein Sapogenin oder einen natürlichen Extrakt, der ein Sapogenin enthält, und mindestens einen Metall-Proteinase-Inhibitor enthält.

2. Zusammensetzung nach Anspruch 1, **dadurch gekennzeichnet, dass** das Sapogenin unter Diosgenin, Hecogenin, Smüagenin und Sarsapogenin ausgewählt ist.

3. Zusammensetzung nach Anspruch 1, **dadurch gekennzeichnet, dass** der natürliche Extrakt unter Boxhornklee und Extrakten von Dioscoreaceae ausgewählt ist.

4. Zusammensetzung nach Anspruch 3, **dadurch gekennzeichnet, dass** der Dioscoreaceae-Extrakt ein Extrakt aus der Wurzel von wildem Yams ist.

5. Zusammensetzung nach einem der vorhergehenden Ansprüche, **dadurch gekennzeichnet, dass** sie 0,001 bis 5 Gew.-% Sapogenin, bezogen auf das Gesamtgewicht der Zusammensetzung, enthält.

6. Zusammensetzung nach einem der vorhergehenden Ansprüche, **dadurch gekennzeichnet, dass** der Metall-Proteinase-Inhibitor ein Metall-Proteinase-Gewebsinhibitor ist.

7. Zusammensetzung nach einem der Ansprüche 1 bis 6, **dadurch gekennzeichnet, dass** der Metall-Proteinase-Inhibitor ein MMP-1-Inhibitor natürlicher Herkunft ist.

8. Zusammensetzung nach Anspruch 7, **dadurch gekennzeichnet, dass** der Metall-Proteinase-Inhibitor das Lycopin ist.

9. Zusammensetzung nach einem der vorhergehenden Ansprüche, **dadurch gekennzeichnet, dass** sie 10⁻¹⁰ bis 2 Gew.-% Metall-Proteinase-Inhibitor, bezogen auf das Gesamtgewicht der Zusammensetzung, enthält.

10. Kosmetische Verwendung der Zusammensetzung nach einem der vorhergehenden Ansprüche zur Vorbeugung oder Behandlung der Anzeichen der Hautalterung.

11. Kosmetische Verwendung der Zusammensetzung nach einem der Ansprüche 1 bis 9 zur Vorbeugung oder Behandlung des Verlustes der Festigkeit und/oder der Geschmeidigkeit der Haut und/oder der Atrophie der Haut und/oder der Bildung von Falten.

12. Kosmetische Verwendung der Zusammensetzung nach einem der Ansprüche 1 bis 9 zur Behandlung der Kopfhaut.

13. Verwendung der Zusammensetzung nach einem der Ansprüche 1 bis 9 zur Herstellung eines Präparats, das zur Behandlung der Kopfhaut vorgesehen ist.

## Claims

1. Composition including, in a cosmetically or dermatologically acceptable medium, a sapogenin or a natural extract comprising it and at least one metalloproteinase inhibitor.

2. Composition according to Claim 1, **characterized in that** said sapogenin is chosen from diosgenin, hecogenin, smilagenin and sarsapogenin.

3. Composition according to Claim 1, **characterized in that** said natural extract is chosen from fenugreek and extracts of Dioscoreae.

4. Composition according to Claim 3, **characterized in that** said extract of Dioscoreae is a wild yam root extract.

5. Composition according to any one of the preceding claims, **characterized in that** it includes from 0.001 to 5% by weight of sapogenin with respect to the total weight of the composition.

6. Composition according to any one of the preceding claims, **characterized in that** said metalloproteinase inhibitor is a tissue inhibitor of metalloproteinases.

7. Composition according to any one of Claims 1 to 6, **characterized in that** said metalloproteinase inhibitor is an MMP-1 inhibitor of natural origin.

8. Composition according to Claim 7, **characterized in that** said metalloproteinase inhibitor is lycopene.

9. Composition according to any one of the preceding claims, **characterized in that** it comprises from 10⁻¹⁰ to 2% by weight of metalloproteinase inhibitor with respect to the total weight of the composition.

10. Cosmetic use of the composition according to any one of the preceding claims, for preventing or treating signs of cutaneous aging.

11. Cosmetic use of the composition according to any one of Claims 1 to 9 for preventing or treating loss of firmness and/or of suppleness of the skin and/or atrophy of the skin and/or the formation of wrinkles.

12. Cosmetic use of the composition according to any one of Claims 1 to 9 in the treatment of the scalp.

13. Use of the composition according to any one of Claims 1 to 9 in manufacturing a preparation intended for the treatment of the scalp.
